(19) Europäisches Patentamt
European Patent Office
Office européen des brevets

(11) **EP 3 809 964 B1**

(12) **EUROPÄISCHE PATENTSCHRIFT**

(45) Veröffentlichungstag und Bekanntmachung des Hinweises auf die Patenterteilung:
**07.06.2023 Patentblatt 2023/23**

(21) Anmeldenummer: **19733442.8**

(22) Anmeldetag: **19.06.2019**

(51) Internationale Patentklassifikation (IPC):
**A61B 5/05** (2021.01)   **A61B 5/113** (2006.01)
**A61B 5/08** (2006.01)

(52) Gemeinsame Patentklassifikation (CPC):
**A61B 5/0507; A61B 5/0816; A61B 5/113**

(86) Internationale Anmeldenummer:
**PCT/EP2019/066257**

(87) Internationale Veröffentlichungsnummer:
**WO 2019/243444 (26.12.2019 Gazette 2019/52)**

(54) **MESSVORRICHTUNG UND VERFAHREN ZUR BESTIMMUNG ZUMINDEST EINES RESPIRATORISCHEN PARAMETERS**

MEASURING DEVICE AND METHOD FOR DETERMINING AT LEAST ONE RESPIRATORY PARAMETER

DISPOSITIF DE MESURE ET PROCÉDÉ SERVANT À DÉFINIR AU MOINS UN PARAMÈTRE RESPIRATOIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.06.2018 DE 102018210051**

(43) Veröffentlichungstag der Anmeldung:
**28.04.2021 Patentblatt 2021/17**

(73) Patentinhaber: **Fraunhofer-Gesellschaft zur Förderung
der angewandten Forschung e.V.
80686 München (DE)**

(72) Erfinder:
- **RINGKAMP, Jan**
  **70569 Stuttgart (DE)**
- **LANGEJÜRGEN, Jens**
  **70569 Stuttgart (DE)**
- **LEBHARDT, Philipp**
  **70569 Stuttgart (DE)**
- **RADLER, Philipp**
  **70569 Stuttgart (DE)**

(74) Vertreter: **Pfenning, Meinig & Partner mbB
Patent- und Rechtsanwälte
Theresienhöhe 11a
80339 München (DE)**

(56) Entgegenhaltungen:
US-A- 4 926 868         US-A- 4 958 638
US-A1- 2008 077 015     US-A1- 2010 106 027
US-A1- 2012 073 574     US-A1- 2013 226 527
US-A1- 2015 031 979     US-A1- 2016 174 926
US-A1- 2016 374 622     US-A1- 2017 172 425

- PETKIE T DOUGLAS: "Millimeter-wave radar systems for biometric applications", SPIE, PO BOX 10 BELLINGHAM WA 98227-0010 USA, Bd. 7485, 17. September 2009 (2009-09-17), Seite 748502, XP040502348,
- SANG-GYU KIM ET AL: "Compact Vital Signal Sensor Using Oscillation Frequency Deviation", IEEE TRANSACTIONS ON MICROWAVE THEORY AND TECHNIQUES, PLENUM, USA, Bd. 60, Nr. 2, 1. Februar 2012 (2012-02-01), Seiten 393-400, XP011398805, ISSN: 0018-9480, DOI: 10.1109/TMTT.2011.2175403

**Beschreibung**

Die Erfindung betrifft eine Messvorrichtung und ein Verfahren zur Bestimmung zumindest eines respiratorischen Parameters, wobei ein elektromagnetisches Feld in einen Körper eingestrahlt wird und auf einer gegenüberliegenden Seite des Körpers empfangen wird, dann eine Phase des empfangenen Wechselfelds mit einer Phase des eingestrahlten Wechselfeldes zeitabhängig verglichen wird und aus einem Ergebnis des Vergleichs der zumindest eine respiratorische Parameter bestimmt wird.

Die Beobachtung der Atemtätigkeit hat eine Vielzahl praktischer Anwendungen. Beispielsweise kommt in einer Vielzahl wissenschaftlicher Untersuchungen der Messung der Atemaktivität, zum Beispiel in besonderen Situationen oder unter sportlicher Belastung, eine zentrale Rolle zu. Derartige Untersuchungen ermöglichen ein besseres Verständnis des Atemverhaltens in Abhängigkeit von äußeren Einflüssen.

Auch für die maschinelle Beatmung von Patienten kann die Beobachtung der Atemtätigkeit von Bedeutung sein. Hier sollte beispielsweise vermieden werden, die künstliche Beatmung gegen eine Spontanatmung durchzuführen.

Ein großer Teil der etablierten Verfahren kombinieren ein Elektrokardiogramm (EKG) mit der Impedanzkardiographie (IKG) und schätzen die kardiale Komponente, ziehen diese vom Impedanzsignal ab, um aus dem resultierenden Signal die respiratorische Komponente zu gewinnen. Da die Erfassung der respiratorischen Parameter hierbei indirekt durch die Schätzung der kardialen Komponente abgeleitet wird, hängt diese zwingend von der Qualität des kardialen Modells ab. Weiterhin wird hierbei hauptsächlich die Atemfrequenz bestimmt. Eine Aussage über Tidalvolumen und Spontanatmung ist nicht direkt möglich.

Ein anderes Verfahren ist die Messung der Dehnung des Brustkorbs und des Abdomens mit Hilfe der respiratorischen induktiven Plethymographie (RIP). Hierbei werden dem Patienten zwei elastische Bänder um Thorax und Abdomen gelegt. In die Bänder sind Spulen eingearbeitet, die bei einer Dehnung der Bänder ihre Induktivität ändern. Diese Methode kann aufgrund der elastischen Bänder unangenehm und einengend auf den Patienten wirken. Ein vergleichbares Verfahren ist auch über Dehnungsmessstreifen vorstellbar, wobei sich keine weiteren Vorteile des Verfahrens ergeben.

Die US 2013/0226527 A1 beschreibt ein System und Verfahren zum Bestimmen physiologischer Parameter basierend auf Impedanzmessungen. Es werden elektrische Meßsignale von einer Vielzahl von Transducern aufgenommen, die mit einer Oberfläche des Objektes gekoppelt sind.

Die US 2017/0172425 A1 beschreibt ein Verfahren zum Bestimmen von Puls- und/oder Atemfrequenz einer Person. Es wird dabei eine Doppler-Radarmessung ausgeführt. Ein Signal wird auf den Körper angestrahlt und eine Frequenzverschiebung des empfangenen Signals gemessen.

Die US 4,958,638 beschreibt ein kontaktloses Messverfahren für Vitalfunktionen. Auch hier wird eine Doppler-Radarmessung mit elektromagnetischen Wellen ausgeführt, die an der Oberfläche des Körpers reflektiert werden.

Die US 2016/0374622 A1 betrifft ein Verfahren, um bei der Detektion von Vitalparametern Bewegungen des Körpers herauszurechnen. Diese Veröffentlichung beschreibt ebenfalls eine Doppler-Radarmessung, wobei elektromagnetische Wellen von beiden Seiten des Körpers auf diesen eingestrahlt werden können. Der Puls beispielsweise führt zu einer Bewegung der Oberfläche des Brustkorbs, die mittels der Radarmessung bestimmt werden kann.

Die US 2008/0077015 A1 beschreibt ein Verfahren zur Bestimmung der Anwesenheit und/oder physiologischen Bewegung von einem oder mehreren Subjekten mittels Doppler-Radar-Systemen mit mehreren Empfängern. Auch hier führt beispielsweise der Puls eine Person zu einer Bewegung der Oberfläche seines Brustkorbs, die mittels der Doppler-Radarmessung detektiert werden kann.

PETKIE T Douglas: "Millimeter-wave radar systems for biometric applications", SPIE, PO BOX 10 Bellingham WA 98227-0010 USA, Bd. 7485, 17. September 2009 (2009-090-17), Seite 748502 beschreibt ein Millimeter-Wellen-Radar-System für biometrische Anwendungen. Auch in dieser Veröffentlichung wird die Messung mittels an der Oberfläche des Körpers reflektierten elektromagnetischen Wellen ausgeführt.

Die US 4,926,868 A beschreibt ein Verfahren und eine Vorrichtung für chemodynamische Messungen. Das Verfahren basiert auf den komplexen Feldamplituden von Mikrowellen. Auch hier wird eine Dopplermessung von Geschwindigkeiten ausgeführt.

Die US 2012/0073574 A1 beschreibt ein Verfahren und ein System zur Detektion von Asynchronität der Atmung. Es wird hierbei ein Signal empfangen, dass in einer Fourier-Transformation analysiert wird. Auf diese Weise wird ein Frequenzspektrum des Signals erhalten, aus dem die gewünschten Größen ableitbar sind.

Die US 2016/0174926 A1 beschreibt ein Verfahren und ein System für adaptive Computertomographie. Es werden dabei Messungen von interessierenden Bereichen ausgeführt, die mit vorgegebenen Schwellenwerten verglichen werden.

Die US 2015/0031979 A1 beschreibt ein Verfahren zur Bestimmung eines oder mehrerer patientenspezifischer Parameter eines dielektrischen Thoraxmodels. Die Modellparameter werden aus elektromagnetischen Messungen am thorakalen Intrakörperbereich und korrespondierenden (z.B. gleichzeitigen) Thoraxvolumenmessungen bestimmt während der Patient Thoraxvolumenmanipulationen durchführt. Das derart kalibrierte dielektrische Thoraxmodell kann dann zur Bestimmung von respiratorischen Parameter aus elektromagnetischen Messungen verwendet werden.

Aufgabe der vorliegenden Erfindung ist es, eine Messvorrichtung und ein Verfahren zur Bestimmung zumindest eines respiratorischen Parameters anzugeben, die eine Bestimmung des entsprechenden Parameters ermöglichen.

Diese Aufgabe wird gelöst durch die Messvorrichtung zur Bestimmung zumindest eines respiratorischen Parameters nach Anspruch 1, das Verfahren zum Messen zumindest eines respiratorischen Parameters nach Anspruch 7, das Beatmungsgerät nach Anspruch 19, das Gerät zur Injektion eines Kontrastmittels nach Anspruch 20 und das Gerät zur Bildgebung nach Anspruch 21.

Die jeweiligen abhängigen Ansprüche geben vorteilhafte Ausgestaltungen der erfindungsgemäßen Messvorrichtung und des erfindungsgemäßen Verfahrens an.

Erfindungsgemäß wird eine Messvorrichtung zur Bestimmung zumindest eines respiratorischen Parameters angegeben. Die Messvorrichtung weist zumindest eine Sendestruktur und zumindest eine Empfangsstruktur auf. Vorteilhafterweise kann die zumindest eine Sendestruktur eine Antenne oder eine Elektrode sein. Vorzugsweise kann die zumindest eine Empfangsstruktur eine Antenne oder eine Elektrode sein.

Erfindungsgemäß ist die zumindest eine Sendestruktur und/ oder die zumindest eine Empfangsstruktur in einer Auflagefläche für einen Patienten oder in einem Seitenelement eines Patientenbettes angeordnet. Vorzugsweise ist zumindest eine Sendestruktur und/ode zumindest eine Empfangsstruktur so ausgestaltet, dass sie auf einem Körper einer Person, eines Tieres oder eines Phantoms an dessen Außenseite anbringbar sind. Die Sendestruktur und/oder die Empfangsstruktur können beispielsweise aufklebbar ausgestaltet sein. Ein elektrisch leitfähiger Kontakt der Sendestruktur und/oder Empfangsstruktur mit dem Körper kann vorteilhaft sein, ist aber nicht notwendig.

Die erfindungsgemäße Messvorrichtung weist zumindest einen Signalgenerator auf, der mit der zumindest einen Sendestruktur elektrisch gekoppelt ist. Durch den Signalgenerator ist eine Wechselspannung erzeugbar, mit der die Sendestruktur beaufschlagbar ist. Auf diese Weise kann durch die Sendestruktur ein elektromagnetisches Wechselfeld erzeugt werden.

Erfindungsgemäß werden die Antennen der Empfangs- und Sendestruktur so angeordnet, dass sie sich wechselseitig im Nahfeld befinden. Die Antenne der Sendestruktur ist dann mit der entsprechenden Antenne der Empfangsstruktur gekoppelt in dem Sinne, dass sich eine gekoppelte Impedanz zwischen den Antennen ausbildet. Diese Impedanz $Z_{12}$ kann für das Beispiel zweier Dipole als Sende- und Empfangsantenne wie folgt beschrieben werden:

$$Z_{12} = \frac{l^2}{4\pi r^2}\left[\underbrace{\frac{1}{j\omega\cdot\varepsilon\cdot r}}_{C} + R_0 + \underbrace{j\omega\cdot\mu\cdot r}_{L}\right]e^{-j2\pi r/\lambda}$$

Dabei überwiegt der Permittivitätsterm, je näher, in Relation zu der Wellenlänge, die Antennen sind (H. Wheeler, "The Radiansphere around a Small Antenna," Proc. IRE, vol. 47, no. 8, pp. 1325-1331, 1959). Beruht die Messung zum Beispiel auf einer Detektion der Permittivtätsänderung, ist es folglich vorteilhaft, eine möglichst große Wellenlänge $\lambda$ zu haben, um das Verhältnis r (Abstand zwischen den Antennen) zu $\lambda$ (Wellenlänge), so klein wie möglich zu halten. Vorteilhafterweise wird eine optimale Wellenlänge (bzw. Frequenz) gewählt, die in Relation zum Körper klein genug ist, um eine deutliche Verschiebung der Phase bei Inspiration und Exspiration sehen zu können, und gleichzeitig groß genug ist, um eine noch signifikante Kopplung im oben genannten Sinne zu erhalten. In einer nicht beanspruchten Ausführung befinden sich die Antennen nicht in ihrem Nahfeld, sind also im oben genannten Sinne nicht gekoppelt.

Werden erfindungsgemäß die Antennen im Nahfeld zueinander angeordnet, so ist erfindungsgemäß der Abstand zwischen den Antennen kleiner oder gleich der 4-fachen Wellenlänge $\lambda$, vorteilhaft kleiner oder gleich der 3-fachen Wellenlänge $\lambda$, vorteilhaft kleiner oder gleich der zweifachen Wellenlänge ist. Auch die Definition des Nahfeldes der IEEE kann optional zugrundegelegt werden. Hier ist die äußere Grenze definiert als der Abstand $\lambda/(2\pi)$ von der Antennenoberfläche, wobei $\lambda$ die Wellenlänge im Freiraum ist (Vgl. IEEE Standard for Definitions of Terms for Antennas, IEEE Std 145-2013). Folglich ist vorteilhaft der Abstand der Antennen des Antennenpaars kleiner oder gleich $\lambda/(2\pi)$.

Vorteilhafterweise wird das gekoppelte System der Sende- und Empfangsstrukturen in der Resonanzfrequenz der Sendeantenne und/oder der Resonanzfrequenz der durch die Empfangsantenne in der Sendeantenne erzeugten Resonanzfrequenz betrieben.

Es ist vorteilhaft, wenn die Sende- und Empfangsantennen jeweils eine gemeinsame Resonanz aufgrund der Kopplung im oben genannten Sinne ausbilden. Besonders vorteilhaft kann die Güte der Resonanz angepasst bzw. verändert werden um an die konkrete Anwendung angepasst zu werden. Hierbei führt eine hohe Güte zu einer hohen Sensitivität bei einem kleineren Dynamikbereich. Entsprechend führt eine geringe Güte zu einer geringen Sensitivität bei einem größeren Dynamikbereich. Dynamik ist hier definiert als der Messbereich, der einem Phasenversatz [0..2Pi] eindeutig zugewiesen werden kann. Erstreckt sich der Phasenversatz über mehr als 2*Pi, so entsprechen die Phasenversätze nicht mehr eindeutig einem Abstand der Antennen. Dies ist nicht notwendig problematisch, da auch am Verlauf des Phasenversatzes erkannt werden kann, welchen Abstand die Antennen haben, vorteilhaft ist es jedoch, die Wellenlänge so zu wählen, dass der Bereich 0 bis 2Pi nicht überschritten wird, wenn die Atemtätigkeit ihre volle Amplitude durchläuft.

So kann sich zum Beispiel eine hohe Güte für eine Anwendung an Neu- oder Frühgeborenen eignen, da nur ein kleiner Volumenbereich gemessen werden muss, allerdings mit einer hohen Auflösung. Eine geringe Güte kann sich für eine Anwendung an Erwachsenen eignen, da ein großer Volumenbereich vermessen werden muss, allerdings eine geringere Auflösung dafür ausreichend ist.

In einer vorteilhaften Ausgestaltung der Erfindung können die Sende- und/oder die Empfangsantenne meanderförmig ausgestaltet sein. Dabei kann die Antenne auf einem Substrat aufgebracht sein, das besonders vorteilhaft eine hohe Permittivität hat. Hierdurch vergrößert sich die elektrische Länge der Antenne im Vergleich zu ihrer mathematischen bzw. geometrischen Länge.

Es ist auch vorteilhaft möglich, keramische Antennen als Sende- und/oder Empfangsantenne einzusetzen, wo die Welle über Keramik läuft. Auch möglich ist die Verwendung von Patchantennen.

Dadurch ist der vermessenen Bereich des Antennenpaars besser definiert und weniger störanfällig für hochpermittive Körper (z.B. Arme oder Hände), die von außen in das Nahfeld der Antenne kommen. Eine solche Richtcharakteristik kann zum Beispiel erzielt werden durch eine mit der Masse verbundene Fläche hinter der Antenne, also distal zum Körper. Alternativ oder zusätzlich kann eine Antennentopologie mit nicht-unipolarer Abstrahlcharakteristik verwendet werden. Es ist vorteilhaft auch möglich, einen niederpermittiven Körper hinter den Antennen, also distal zum Körper, einzubringen. Eine weitere Möglichkeit ist es, ein Absorbermaterial distal zum Körper anzuordnen.

In einer vorteilhaften Ausgestaltung können die Antennen differenziell kontaktiert werden. Hierzu können die Antennen zum Beispiel mit einem verdrillten Leiterpaar mit zwei Strömen kontaktiert werden, wobei die Ströme im 180° Phasenversetzt sind.

Die Antennen können im allgemeinen auch beabstandet zum Körper angeordnet werden. Insbesondere können sich dielektrische oder isolierende Materialien zwischen den Antennen und dem Körper befinden. Ein expliziter Sonderfall hiervon ist, dass sich Luft zwischen den Antennen und dem Körper befindet.

In einer nicht beanspruchten Ausführung werden zwei isolierte Leiter um den Thorax des Patienten gelegt, zum Beispiel als umlaufender Leiter. Dabei wird ein Leiter ventral z.B. über den Brustbereich und ein Leiter dorsal z.B. über den Rückenbereich geführt. Der Thorax des Patienten kann somit wie ein Dielektrikum zwischen einem Leiterpaar angeordnet sein. Aufgrund der Atmung ändert sich die Permittivität des Thorax und damit die Laufzeit der elektromagnetischen Wellen, die leitergeführt durch den Thorax läuft. Dies kann als Änderung der Phase über der Zeit detektiert werden. Hierbei ist es von Vorteil, das Leiterpaar differentiell anzulegen, das heißt, dass an einem Leiter die Spannung steigt, während am zweiten Leiter die Spannung in gleichem Maße abnimmt und umgekehrt, sobald sich das Wechselfeld umpolt. Zusätzlich ist es von Vorteil die Leiter einseitig zu schirmen, um Störeinflüsse von außen zu reduzieren.

Es ist in einer vorteilhaften Ausgestaltung auch möglich, über die Dämpfung der Welle (sowohl transmittiert (S21 Parameter) als auch reflektiert (S11 Parameter) eine Aussage über die Atmung (Inspiration oder Exspiration) zu treffen. Dabei kann neben der Phaseninformation die Dämpfung als zusätzlicher Parameter gemessen werde, der die Bestimmung der respiratorischen Parameter unterstützt oder die Dämpfung eigenständig mit der erfindungsgemäßen Vorrichtung zu detektieren und für die Bestimmung wenigstens eines respiratorischen Parameters zu verwenden.

Die erfindungsgemäße Messvorrichtung weist außerdem eine Vergleichseinheit auf, mit der als Vergleich eine Differenz der Phase eines von der Empfangsstruktur gelieferten Signals mit einer Phase der Wechselspannung bildbar ist, mit der die Sendestruktur beaufschlagt wird. Dass die Empfangsstruktur das Signal liefert kann dabei optional so verstanden werden, dass die Empfangsstruktur das Signal erzeugt, wobei aber die Empfangsstruktur kein aktives Element sein muss. Es kann auch so verstanden werden, dass das Signal von der Empfangsstruktur empfangen wird, in dem Sinne, dass das Signal von der Empfangsstruktur ausgehend empfangen wird. Die Vergleichseinheit kann hierzu mit der Empfangsstruktur elektrisch gekoppelt sein. Vorteilhaft ist auch ein Vergleich der Phase zwischen von zumindest zwei Empfangsstrukturen empfangenen Signalen möglich.

Erfindungsgemäß weist die Messvorrichtung außerdem eine Auswerteeinheit auf, mit der aus einem Ergebnis des Vergleichs der Phase des von der Empfangsstruktur empfangenen Signals mit der Phase der Wechselspannung und/oder der Phase eines durch die Sendestruktur erzeugten elektromagnetischen Wechselfelds zumindest ein respiratorischer Parameter bestimmbar ist.

Die Messvorrichtung ist vorzugsweise eingerichtet, ein erfindungsgemäßes Verfahren zum Messen zumindest eines respiratorischen Parameters auszuführen. In diesem Verfahren wird über zumindest eine Sendestruktur ein elektromagnetisches Wechselfeld in einen Körper, beispielsweise einer Person, eines Tieres oder eines Dummies eingestrahlt, indem durch einen Signalgenerator eine Wechselspannung erzeugt wird, mit der die zumindest eine Sendestruktur beaufschlagt wird. Das in den Körper eingestrahlte Wechselfeld wird auf einer, vorzugsweise gegenüberliegenden, Seite des Körpers durch zumindest eine Empfangsstruktur empfangen, nachdem es den Körper durchlaufen hat. Die gegenüberliegende Seite kann hierbei vorzugsweise jene Seite sein, die jener Seite, von der das elektromagnetische Wechselfeld eingestrahlt wird, bezüglich der Körperachse gegenüberliegt. Es wird dann in einem Vergleichsschritt eine Differenz der Phase eines von der zumindest einen Empfangsstruktur empfangenen Signals mit einer Phase der Wechselspannung zeitabhängig gebildet und aus einem Ergebnis dieses Vergleichs zumindest ein respiratorischer Parameter bestimmt. Es ist optional auch möglich, Sende- oder Empfangsstruktur am Thorax links und entsprechend Empfangs- oder Sendestruktur am Abdomen links anzuordnen oder die Sende- oder Empfangsstruktur am Thorax links und entsprechend die Empfangs- oder Sendestruktur am Abdomen rechts anzuordnen, um das Signal durch Lunge und Zwerchfell laufen zu lassen. Erfindungsgemäß ist die Sende- und/oder Empfangsstruktur in einer Auflagefläche für einen Patienten oder in einem Seitenteil eines Patientenbettes angeordnet.

Die Sendestruktur kann mit dem Signalgenerator vorteilhaft über ein Kabel verbunden sein. In diesem Fall wird vorzugsweise die Phase der Wechselspannung an einem Widerstand gemessen, über den das Kabel gespeist wird. Ebenso kann die Empfangsstruktur mit der Vergleichseinheit über ein Kabel verbunden sein. Es kann dann vorteilhaft die Phase des von der Empfangsstruktur empfangenen Signals an einem Abschlusswiderstand gemessen werden, der vorteilhaft zwischen der Vergleichseinheit und einem Bezugspotential angeschlossen ist. Vorteilhaft werden die Phase des Ausgangs des Signalgenerators oder ein dazu definierter oder zeitlich konstanter Bezug mit der Phase des empfangsseitigen Eingangs der Vergleichseinheit oder einem dazu definierten oder zeitlich konstanten Bezug verglichen.

Dass die Phase des von der Empfangsstruktur empfangenen Signals mit der Phase der Wechselspannung oder des eingestrahlten Wechselfelds verglichen wird, bedeutet, dass eine Differenz dieser Phasen gebildet wird.

Dabei ist diese Phasendifferenz zwischen der Phase des empfangenen Signals und der Phase der Wechselspannung oder des eingestrahlten Signals zeitabhängig bestimmbar. Somit wird diese Phasendifferenz also für mehrere nicht zusammenfallende Zeitpunkte bestimmt und dann der respiratorische Parameter aus einem zeitlichen Verlauf der Phasendifferenz bestimmt.

Vorteilhafterweise kann aus dem Vergleich der Phasen von Wechselspannung und empfangenen Signal auf eine Laufzeit der elektromagnetischen Welle durch den Körper geschlossen werden. Auch solche Laufzeiten werden vorzugsweise zeitabhängig bestimmt, so dass der zumindest eine respiratorische Parameter aus dem zeitlichen Verlauf der Laufzeit ermittelt werden kann.

Die Phase bzw. die Laufzeit korrespondiert mit der aktuellen Ausdehnung des Körpers zwischen der Sendestruktur und der Empfangsstruktur sowie gegebenenfalls einer Änderung von dielektrischen Eigenschaften des Volumens zwischen Sende- und Empfangsstruktur. Bevorzugterweise wird das elektromagnetische Wechselfeld über die zumindest eine Sendestruktur in einen Thorax und/oder ein Abdomen des Körpers eingestrahlt. Hierzu kann die zumindest eine Sendestruktur auf dem Thorax bzw. dem Abdomen angebracht werden. Es wird dann das Wechselfeld durch die zumindest eine Empfangsstruktur vorzugsweise auf der gegenüberliegenden Seite des Thorax und/oder des Abdomens empfangen. Besonders bevorzugt werden die Sendestruktur und die Empfangsstruktur dabei lateral am Abdomen und/oder am Thorax angebracht, so dass die elektromagnetische Welle den Körper lateral durchläuft.

In diesen Fällen korrespondiert die Phase bzw. die Laufzeit mit der aktuellen Ausdehnung der Lunge (incl. Zwerchfell, Rippenbögen, usw.). Ändert sich durch Atmung die Ausdehnung der Lunge, so ändert sich auch die Phasendifferenz bzw. die Laufzeit. Die Änderung der Phasendifferenz bzw. die Änderung der Laufzeit beruht hierbei zum einen auf der Dehnung des Körpers bzw. des Brustkorbs bei der Inspiration, zum anderen auf der Änderung der dielektrischen Eigenschaften des von der elektromagnetischen Welle durchlaufenden Bereichs des Körpers. Zum einen verschieben sich im Körper Gewebe und Organe und zum anderen ändert sich der Luftgehalt der Lunge, was die elektrischen Eigenschaften verändert.

Vorteilhafterweise kann, beispielsweise über einen Phasenhub, d. h. die Differenz zwischen der minimalen Phasendifferenz und der maximalen Phasendifferenz in einem gewählten Zeitfenster, eine Atemtiefe der Person, des Tieres oder des Phantoms abgeleitet werden. In diesem Falle kann der zumindest eine respiratorische Parameter ein Atemzugvolumen der Person, des Tieres oder des Dummies sein.

In einer vorteilhaften Ausgestaltung der Erfindung kann das eingestrahlte Wechselfeld bzw. die an die Sendestruktur angelegte Wechselspannung mit variierbarer und/oder variierender Frequenz erzeugt werden. Vorteilhafterweise kann dann der zumindest eine respiratorische Parameter bei einer der Mehrzahl der Frequenzen bestimmt werden, bei welcher die Kopplung zwischen dem eingestrahlten Wechselfeld und dem empfangenen Wechselfeld bzw. zwischen der Sendestruktur und der Empfangsstruktur maximal ist und/oder bei welcher eine Amplitude des empfangenen Wechselfelds oder des von der Empfangsstruktur empfangenen Signals maximal ist. Es ist auch möglich, jene Frequenz für die Bestimmung des respiratorischen Parameters zu wählen, bei der sich über den Verlauf eines Atemzyklusses eine maximale Veränderung der Phasendifferenz zwischen der Phase des von der Empfangsstruktur empfangenen Signals und der Phase der Wechselspannung ergibt. Auf diese Weise kann eine optimale Messfrequenz ermittelt werden. Die optimale Messfrequenz kann, insbesondere abhängig von der Körpergröße, für verschiedene Personen, Tiere oder Phantome unterschiedlich sein.

Vorteilhaft ist es auch möglich, bei mehreren Frequenzen parallel zu vermessen und die bei den verschiedenen Frequenzen bestimmten Werte des zumindest einen respiratorischen Parameters miteinander zu vergleichen oder miteinander zu verrechnen, beispielsweise mittels einer Durchschnittsbildung. Ferner ist es optional möglich, in einem Zeitmultiplex die Frequenzen in kurzer zeitlicher Abfolge zu senden oder in einem Frequenzmultiplex die Frequenzen zeitgleich zu senden und die einzelnen Messfrequenzen zur Plausibilitätsprüfung, beispielsweise über die Bewertung ihrer zeitlichen Gradienten, zu verwenden.

Diese Plausibilitätsprüfung kann unter anderem verwendet werden, um Bewegungsartefakte aus der Messreihe auszuschließen. Ferner ist es optional möglich die unterschiedlichen Messfrequenzen über adaptive Filtertechniken, Korrelationsfilter oder Verfahren des maschinellen Lernens zur Verbesserung der Signalqualität zu verwenden.

In einer vorteilhaften Ausgestaltung der Erfindung kann die zumindest eine Sendestruktur über ein erstes Kabel mit dem Signalgenerator verbunden sein. Dabei hat das erste Kabel vorzugsweise eine vorgegebene Wellenimpedanz. Bevorzugterweise wird dann die Sendestruktur über einen ersten Widerstand gespeist, dessen Wert gleich der Wellenimpedanz des ersten Kabels ist. Vorteilhafterweise ist die zumindest eine Empfangsstruktur über ein zweites Kabel mit der Vergleichseinheit verbunden, wobei das zweite Kabel vorzugsweise eine vorgegebene Wellenimpedanz hat. Bevorzugterweise wird dann die Empfangsstruktur über einen zweiten Widerstand terminiert, dessen Wert gleich der Wellenimpedanz des zweiten Kabels ist. Beispielsweise können der erste und der zweite Widerstand sowie die Wellenimpedanz des ersten und des zweiten Kabels 50 Ohm betragen. Optional kann auch die Wellenimpedanz der Sendestruktur gleich der Wellenimpedanz des Kabels sein. Gleiches gilt vorzugsweise für die Wellenimpedanz der Empfangsstruktur.

Vorteilhafterweise wird die Wechselspannung und/oder das eingestrahlte Wechselfeld mit einer Frequenz von größer oder gleich 10 MHz, vorzugsweise größer oder gleich 30 MHz, vorzugsweise größer oder gleich 100 MHz und/oder kleiner gleich 1.000 MHz, vorzugsweise kleiner oder gleich 500 MHz, vorzugsweise kleiner oder gleich 300 MHz erzeugt.

In einer vorteilhaften Ausgestaltung der Erfindung können zwei, drei, vier oder mehr als vier Sendestrukturen vorgesehen sein und/oder zwei, drei, vier oder mehr als vier Empfangsstrukturen. Auf diese Weise lassen sich genauere Messergebnisse erzielen. Für den Fall, dass mehrere Sendestrukturen und mehrere Empfangsstrukturen eingesetzt werden, ist ein zeitlicher Multiplex aller Sendestrukturen möglich, um eine optimale Sendestruktur-Empfangs-struktur-Paarung zu finden.

Für den Fall, dass eine Sendestruktur und mehrere Empfangsstrukturen vorgesehen werden, können die Ergebnisse, die durch die Signale der unterschiedlichen Empfangsstrukturen ermittelt werden, verwendet werden, um die Plausibilität des Ergebnisses zu prüfen. Ist beispielsweise ein Gradient auf allen Empfängern sehr hoch, kann dies bedeuten, dass der vermessene Körper in Bewegung ist. In diesem Fall kann die Messung verworfen werden. Derartige Plausibilitätsprüfungen können im Fall mehrere Sender auch im zeitlichen Multiplex erfolgen. Wird die zeitliche Auflösung der Bestimmung der Phasendifferenz hinreichend hoch gewählt, kann auch ein zeitlicher Versatz der unterschiedlichen Empfangssignale betrachtet werden. Ändert sich die zeitliche Relation der einzelnen Messwerte sehr stark, kann dies ebenfalls auf eine Bewegung des Körpers hindeuten. Ferner ist es optional möglich die unterschiedlichen Empfangssignale über adaptive Filtertechniken, Korrelationsfilter oder Verfahren des maschinellen Lernens zur Verbesserung der Signalqualität zu verwenden.

Als besonders vorteilhaft hat sich eine Kombination zweier Sendestrukturen mit zwei Empfangsstrukturen, eine Kombination einer Sendestruktur mit drei Empfangsstrukturen und eine Kombination einer Sendestruktur mit zwei Empfangsstrukturen erwiesen. Werden beispielsweise zwei Sendestrukturen und zwei Empfangsstrukturen eingesetzt, kann ein Multiplexen zwischen den beiden Paaren vorteilhaft sein. Eine Sendestruktur und mehrere Empfangsstrukturen sind vor allem für die beschriebenen Plausibilitätsprüfungen vorteilhaft, da die Signale der verschiedenen Empfangsstrukturen miteinander verglichen werden können.

In einer vorteilhaften Ausgestaltung der Erfindung kann als ein respiratorischer Parameter bestimmt werden, ob eine Inspiration oder eine Exspiration stattfindet. Hierzu kann die Steigung der Phasendifferenz zwischen der Phase des empfangenen Signals und des eingestrahlten Wechselfelds bestimmt werden, beispielsweise als Ableitung der Phasendifferenz nach der Zeit. Das Vorzeichen der Steigung gibt an, ob eine Inspiration oder eine Exspiration stattfindet. Auch die Bestimmung von Abfolgen von lokalen Minima in der Phasendifferenz und lokalen Maxima in der Phasendifferenz kann verwendet werden, um zu bestimmen, ob eine Inspiration oder eine Exspiration vorliegt. Folgt beispielsweise ein lokales Maximum auf ein lokales Minimum, so kann geschlossen werden, dass eine Inspiration vorliegt. Folgt ein lokales Minimum auf ein lokales Maximum, so kann geschlossen werden, dass eine Exspiration vorliegt. Diese beispielhafte Bestimmung kann in analogen Beispielen analog für andere Bezugsrichtungen ausgeführt werden und zum Beispiel auch davon abhängig gemacht werden, wie viele Wellenlängen in den Körper passen. Ein Beginn der Inspiration oder Exspiration kann beispielsweise durch Bildung der Ableitung der Phasendifferenz nach der Zeit, durch Averaging, Korrelation usw. der steigenden bzw. fallenden Flanke bestimmt werden.

Auch eine Atemfrequenz kann als respiratorischer Parameter bestimmt werden. Diese kann beispielsweise aus dem zeitlichen Abstand zwischen zwei maximalen Phasendifferenzen oder zwei minimalen Phasendifferenzen bestimmt werden, wobei die Atemfrequenz gerade das Inverse des zeitlichen Abstands ist. Ebenso ist es möglich, den zeitlichen Abstand zwischen minimaler/maximaler zu maximaler/minimaler Phasendifferenz zu bestimmen. Der zeitliche Abstand entspricht hierbei der Zeitdauer der jeweiligen Atemphasen. Auch ist es vorteilhaft möglich, die Atemfrequenz über eine Fourier-Transformation aus dem Zeitsignal zu bestimmen. Hierbei muss nur aus dem über die Fourier-Transformation bestimmten Spektrum die Frequenz mit dem größten Betrag ermittelt werden.

Auch ein Tidalvolumen kann als respiratorischer Parameter bestimmt werden. Das Tidalvolumen ist korreliert zu der Differenz zwischen dem lokalen Minimum und dem lokalen Maximum der Phasendifferenz. Im einfachsten Fall kann die Korrelation des Tidalvolumens zu dieser Differenz als linear angenommen werden. Für genauere Bestimmungen kann die Funktion zwischen Tidalvolumen und der besagten Differenz analytisch angenähert oder experimentell vermessen werden.

In einer vorteilhaften Ausgestaltung der Erfindung kann das erfindungsgemäße Verfahren verwendet werden, um eine maschinelle Beatmung zu steuern. Durch eine maschinelle Beatmung wird ein regelmäßiger Atemzyklus herbeigeführt, was bedeutet, dass die Differenz zwischen der Phase des eingestrahlten Wechselfeldes und des empfangenen Wechselfeldes regelmäßig verläuft. Führt die maschinell beatmete Person eine Spontanatmung aus, wird dieser regelmäßige Zyklus in charakteristischer Weise unterbrochen. Vorteilhafterweise kann dann das erfindungsgemäße Verfahren verwendet werden, um dem Beatmungsgerät ein Signal zu senden, das die Inspiration oder Exspiration einsetzt. Das Beatmungsgerät kann dann entsprechend eine vorteilhaft unterstützende Inspiration (Belüftung) oder Exspiration (Unterbrechung der maschinellen Belüftung) starten, so dass sie die Person unterstützt und nicht gegen die Spontanatmung beatmet. Die unterschiedlichen Empfangssignale können beispielsweise über adaptive Filtertechniken, Korrelationsfilter oder Verfahren des maschinellen Lernens verrechnet werden.

Das erfindungsgemäße Verfahren oder die erfindungsgemäße Vorrichtung kann auch in einer Vorrichtung zur Injektion von beispielsweise Kontrastmittel (z.B. für CT / MRT / Ultraschall) verwendet werden, um die Injektion des Kontrastmittels optimal an die Atmung anzupassen. Hierbei kann die Injektion vorteilhaft dann erfolgen, wenn der Patient gerade mit der Exspiration beginnt oder mit der Inspiration beginnt oder den Atem anhält.

Das erfindungsgemäße Verfahren oder die erfindungsgemäße Vorrichtung kann auch für ein bildgebendes Verfahren, z.B. CT, X-RAY- CBCT, MRT, Ultraschall verwendet werden, um während der Aufnahme des Bildes die respiratorischen Parameter (z.B. Inspiration, Exspiration, momentane Thorax- und/oder Abdomendehnung) aufzuzeichnen. Hierdurch kann z.B. eine Fusion der verschiedenen Teilaufnahmen bei einem scannenden bildgebenden Verfahren dahingehend optimiert werden, dass entweder nur Teilaufnahmen bei gleichem respiratorischen Parameter zu einem Gesamtbild zusammengefügt werden oder die Aufnahme eines Teilbildes nur bei geeignetem respiratorischen Parameter erfolgt (z.B. nur am Ende der Exspiration oder am Ende der Inspiration oder zu einem definierten Zustand) oder der zeitlich Verlauf der respiratorischen Parameter während der Teil- und/oder Gesamtaufnahme zur Korrektur der Bilddaten verwendet wird oder zu einer kombinierten Auswertung verwendet wird.

In einer vorteilhaften Ausgestaltung der Erfindung kann das Wechselfeld, vorzugsweise zeitlich nacheinander, mit einer Vielzahl unterschiedlicher Frequenzen angelegt werden. Es kann dann vorteilhaft der zumindest eine respiratorische Parameter aus einem Vergleich der Phase des empfangenen Wechselfeld mit der Phase des eingestrahlten Wechselfelds bei zumindest zweien der angelegten Frequenzen bestimmt werden. Die so erhaltenen Ergebnisse können miteinander verglichen und/oder miteinander verrechnet werden, um einen endgültigen Wert des betreffenden respiratorischen Parameters zu erhalten.

In einer vorteilhaften Ausgestaltung der Erfindung kann eine zeitliche Änderung der Differenz zwischen der Phase des empfangenen Wechselfelds und der Phase des eingestrahlten Wechselfelds in Abhängigkeit von der Zeit dargestellt werden, beispielsweise in einer grafischen Darstellung. Diese ermöglicht es, eine Veränderung des zumindest einen respiratorischen Parameters über die Zeit zu beobachten. Auf diese Weise können beispielsweise Langzeittests (zum Beispiel zur Beobachtung eines Trends) ausgeführt werden und ein direktes Feedback übermittelt werden.

Bevorzugterweise wird die Wechselspannung so an die Sendestruktur angelegt, dass ein eventuell durch den Körper fließender Strom kleiner oder gleich dem zulässigen Patientenhilfsstrom ist, beispielsweise kleiner oder gleich 100 μA.

In einer vorteilhaften Ausgestaltung der Erfindung kann zumindest eine weitere Elektrode und/oder zumindest eine weitere Messfrequenz verwendet und ausgewertet werden, um zumindest eine Störgröße, wie beispielsweise einen Einfluss eines Herzschlags und/oder eine Bewegung des Körpers zu bestimmen und aus dem respiratorischen Parameter herauszurechnen. So kann beispielsweise der Einfluss des Herzschlags mit Hilfe eines Scaled Fourier Linear Combiners oder mit Hilfe anderer adaptiver Filtertechniken aus dem Signal extrahiert werden. Es ist vorteilhaft auch möglich, zumindest eine Störgröße aus dem in dem Verfahren bestimmten zumindest einen respiratorischen Parameter herauszurechnen, beispielsweise mittels eines Frequenzfilters, eines adaptiven Filters, eines Korrelationsfilters und/oder eines Glättungsfilters und/oder einer Ableitung des Signals. Möglich ist hier beispielsweise ein gleitender Mittelwert oder andere Glättungsmethoden wie beispielsweise Savitzky-Golay.

Die gemessenen Signale können auch mit anderen Messsignalen korreliert werden, wie beispielsweise solchen von einem Beatmungsgerät oder dessen Peripherie gelieferten bzw. gemessenen Werten vom momentanen Beatmungszustand (Inspiration, Exspiration), Druck und/oder Fluss.

Das erfindungsgemäße Verfahren kann in vielen vorteilhaften Ausgestaltungen als nicht-diagnostisches Verfahren ausgeführt werden. Es kann beispielsweise verwendet werden, um das Atemverhalten wissenschaftlich besser zu verstehen. Es kann hierzu mittels des erfindungsgemäßen Verfahrens das Atemverhalten bzw. der zumindest eine respiratorische Parameter beobachtet werden, während die Testperson bestimmte Aufgaben ausführt oder bestimmten Belastungen unterworfen wird. In einer vorteilhaften Anwendung des erfindungsgemäßen Verfahrens kann der Körper auch der Körper eines Phantoms sein, wie er beispielsweise für Beatmungsübungen in Erste-Hilfe-Kursen oder in Crash-Tests verwendet wird. Mittels des erfindungsgemäßen Verfahrens können an solchen Körpern die Wirkung von Beatmungstätigkeiten oder von außen wirkenden Kräften untersucht werden.

Erfindungsgemäß wird außerdem ein Beatmungsgerät angegeben, das eingerichtet ist, ein Verfahren wie vorstehend beschrieben auszuführen und dann die Beatmung anhand des zumindest einen respiratorischen Parameters zu steuern. Ein solches Beatmungsgerät kann beispielsweise auch beim Tauchen verwendet werden.

Im Folgenden soll die Erfindung anhand einiger Figuren beispielhaft erläutert werden. Gleiche Bezugszeichen kennzeichnen dabei gleiche oder entsprechende Merkmale. Die in den Beispielen beschriebenen Merkmale können auch unabhängig vom konkreten Beispiel realisiert sein und zwischen den Beispielen kombiniert werden.

Es zeigt

Figur 1    einen prinzipiellen Aufbau einer erfindungsgemäßen Messvorrichtung als Blockschaltbild,

Figur 2    einen Verlauf einer Phase eines empfangenen Signals bei verschiedenen Messfrequenzen,

Figur 3    einen Phasenhub für verschiedene Atemvolumina,

Figur 4    beispielhaft die Bestimmung einer Phasendifferenz zwischen eingestrahltem und empfangenem Signal sowie eine beispielhafte Bestimmung respiratorischer Parameter,

Figur 5    eine Phasendifferenz über die Zeit bei freier und obstruierter Atmung,

Figur 6    eine meanderförmige Antennenstruktur,

Figur 7    die in Figur 6 gezeigte Antennenstruktur in einer Seitenansicht,

Figur 8    eine Ausgestaltung von Sende- und Empfangsstruktur als umlaufende Leiter, und

Figur 9    eine Ausgestaltung von Sende- und Empfangsstruktur beabstandet vom Patienten

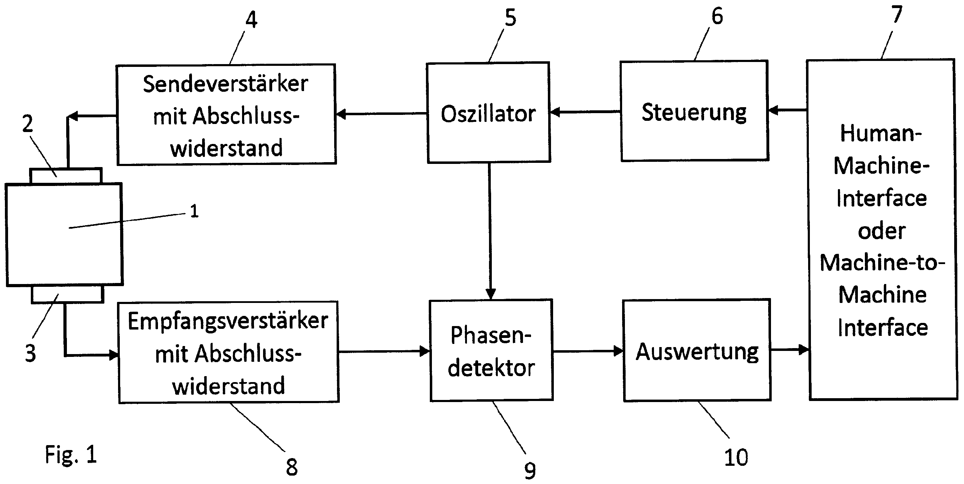

Figur 1 zeigt einen beispielhaften Aufbau einer erfindungsgemäßen Messvorrichtung als Blockschaltbild. Es wird dabei ein respiratorischer Parameter bestimmt, indem ein Körper 1 mit einem elektromagnetischen Wechselfeld durchstrahlt wird. Das elektromagnetische Wechselfeld wird dabei durch eine Sendestruktur 2, die beispielsweise eine Antenne oder eine Elektrode sein kann, in den Körper 1 eingestrahlt und durch eine Empfangsstruktur 3, die ebenfalls eine Antenne oder eine Elektrode sein kann, empfangen. Zur Erzeugung des einzustrahlenden Signals wird die Sendeelektrode 2 mit einer Wechselspannung beaufschlagt, die hier über ein Kabel, beispielsweise ein Koaxialkabel, zugeführt wird. Ein solches Kabel kann eine definierte Wellenimpedanz haben. Die Wechselspannung wird hierbei verstärkt durch einen Sendeverstärker 4 mit einem Abschlusswiderstand, wobei vorzugsweise Wert des der Abschlusswiderstandes gleich der Wellenimpedanz des Kabels ist, über das die Sendeelektrode 2 mit dem Sendeverstärker 4 verbunden ist.

Die Wechselspannung wird dem Sendeverstärker 4 über einen Oszillator 5 zugeführt, der die Wechselspannung mit einer gegebenen Frequenz und einer bestimmten Phase erzeugt. Der Oszillator 5 wird hierzu gesteuert von einer Steuerung 6, die durch eine geeignete Schnittstelle, beispielsweise ein Human-Machine-Interface oder ein Machine-to-Machine Interface steuerbar ist.

Das von der Sendeelektrode 2 in den Körper 1 eingestrahlte Wechselfeld wird durch eine Empfangsstruktur 3 empfangen, die, beispielsweise über ein Koaxialkabel, mit einem Empfangsverstärker 8 mit Abschlusswiderstand verbunden ist. Vorzugsweise hat der Abschlusswiderstand einen Betrag, der gleich ist einer Wellenimpedanz des Kabels, über das der Empfangsverstärker 8 mit der Empfangselektrode 3 verbunden ist. Der Empfangsverstärker 8 ist mit einem Phasendetektor 9 verbunden, der eine Phase des durch die Empfangsstruktur 3 empfangenen und durch den Empfangsverstärker 8 verstärkten Signals messen kann. Der Phasendetektor 9 ist außerdem mit dem Oszillator 5 verbunden, der das Sendesignal erzeugt. Vom Oszillator 5 erhält der Phasendetektor 9 Informationen über die Phase des eingestrahlten Signals. Der Phasendetektor 9 kann daher einen Vergleich der Phase des eingestrahlten Signals mit der Phase des detektierten Signals durchführen und erfindungsgemäß eine Phasendifferenz zwischen diesen Signalen bestimmen. Erfindungsgemäß wird diese Phasendifferenz zeitabhängig bestimmt, also für zumindest zwei oder mehr Zeitpunkte. Der Phasendetektor kann dann die, vorzugsweise zeitabhängige, Phasendifferenz an eine Auswerteeinheit 10 geben, die aus der Phasendifferenz den zumindest einen respiratorischen Parameter bestimmt. Die Auswerteeinheit 10 kann hierzu geeignete Rechen- oder Korrekturschritte ausführen, wie beispielsweise Mittelwertbildung, Ableitung, Bestimmung von Minima und Maxima, adaptive Filterung, Korrelationsfilterung, Frequenzfilterung und dergleichen. Die Auswerteeinheit 10 kann dann das ermittelte Ergebnis, d. h. den respiratorischen Parameter, an die Schnittstelle 7 weitergeben, wo sie für eine Person oder eine Maschine, wie beispielsweise ein Beatmungsgerät, zugänglich ist.

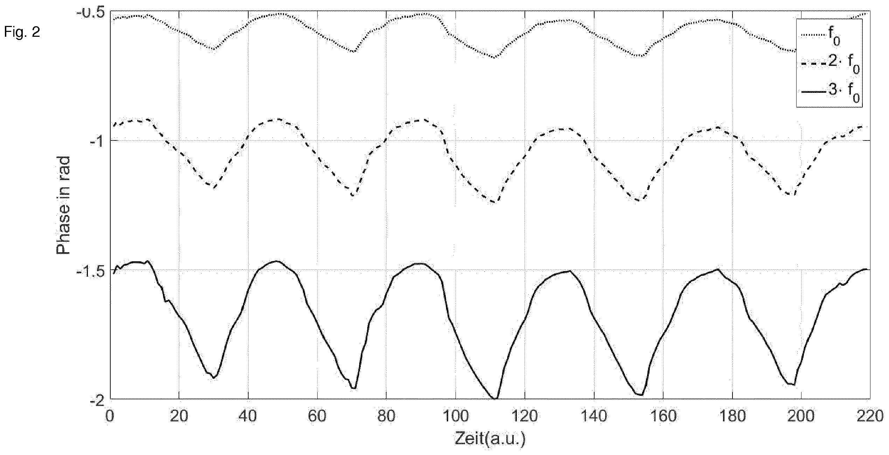

Figur 2 zeigt den Verlauf der Phase über die Zeit während einer natürlichen Atmung bei drei verschiedenen Messfrequenzen, die als gepunktete, gestrichelte und durchgezogene Linie dargestellt sind. Die gestrichelte Linie zeigt dabei eine Messung bei der doppelten Frequenz der gepunkteten Linie und die durchgezogene Linie zeigt eine Messung bei der dreifachen Frequenz der gepunkteten Linie. Zu erkennen ist, dass die Messung bei der dreifachen Frequenz der gepunkteten Linie den größten Phasenhub zeigt und daher als Messfrequenz am besten geeignet ist.

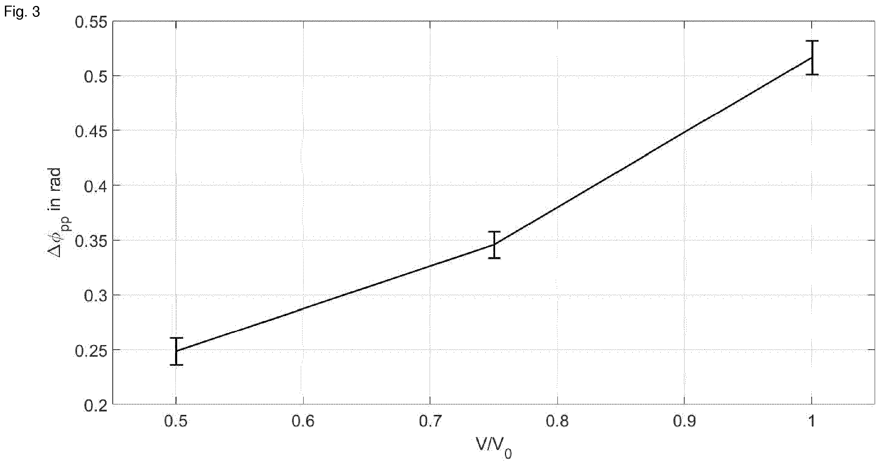

Figur 3 zeigt den Phasenhub für verschiedene Atemvolumina. Dabei ist der Phasenhub auf der vertikalen Achse aufgetragen und die Atemvolumina relativ zu einem Bezugsvolumen auf der horizontalen Achse. Es ist zu erkennen, dass es einen annähernd proportionalen Zusammenhang zwischen dem Atemvolumen und der Phasenverschiebung gibt. Je größer das Atemvolumen, desto größer ist die Phasenverschiebung.

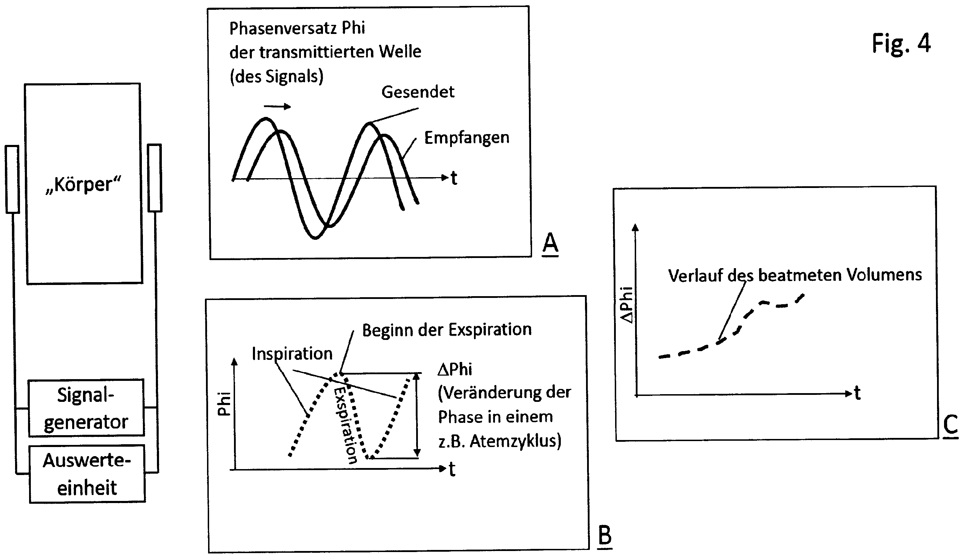

Figur 4 zeigt beispielhaft, wie aus einem gesendeten und einem empfangen Signal ein Verlauf des Atemvolumens bestimmbar ist. In Teilfigur 4A ist dabei der zeitliche Verlauf des gesendeten Signals (größere Amplitude) und des empfangenen Signals (kleinere Amplitude) aufgetragen. Zwischen dem gesendeten und dem empfangenen Signal besteht ein Phasenversatz Phi, der gerade die zeitliche Differenz zwischen gleichen Phasen wie beispielsweise dem Maximum oder dem Minimum des gesendeten und des empfangenen Signals ist multipliziert mit de Kreisfrequenz ist.

Dieser Phasenversatz Phi, auch als Phasendifferenz bezeichnet, ist in Teilfigur 4B gegen die Zeit in Sekunden aufgetragen. Es ergibt sich ein Verlauf des Phasenversatzes wie durch die gepunktete Linie gezeigt. Hier beispielhaft dargestellt steigt der Phasenversatz während der Inspiration an. Während einer Exspiration verringert sich der Phasenversatz. Das Inverse des Abstands bei benachbarter Maxima oder Minima ist gerade die Atemfrequenz.

Figur 4C zeigt einen zeitlichen Verlauf des Phasenversatzes bzw. der Phasendifferenz gegenüber der Zeit. Aufgetragen ist hier ΔPhi, was die Differenz zwischen dem maximalen Phasenversatz Phi und dem minimalen Phasenversatz Phi ist, wie in Figur 4B eingezeichnet. Da sich das Atemvolumen über die Zeit vergrößert, vergrößert sich ΔPhi mit der Zeit.

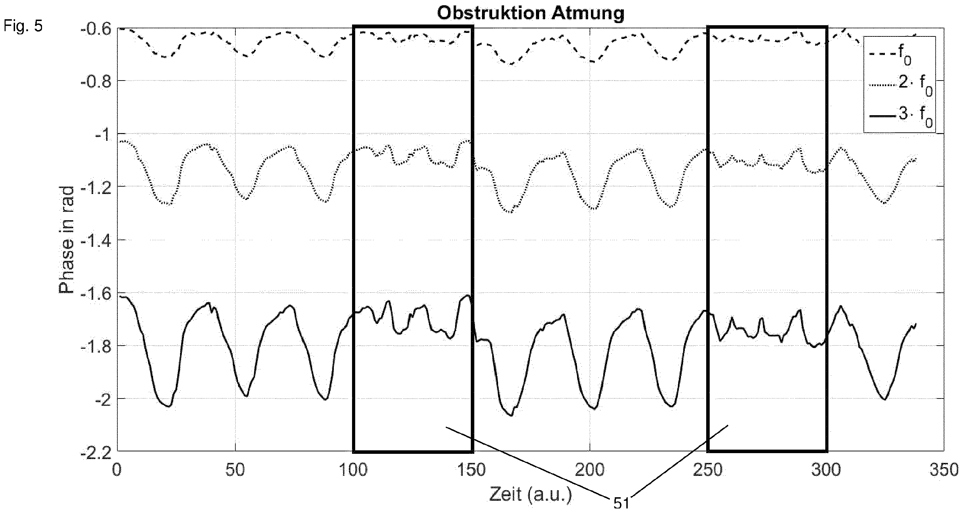

Figur 5 zeigt den Verlauf einer im erfindungsgemäßen Verfahren gemessenen Phase über die Zeit mit Abschnitten freier und obstruierter Atmung. Ein obstruierter Phasenzug ist in den mit 51 gekennzeichneten Abschnitten zu erkennen. Der Phasenhub, also die Differenz zwischen der maximalen Phasendifferenz und der minimalen Phasendifferenz ist hier während eines Atemzugs deutlich kleiner als im Falle der freien Atmung. Auf diese Weise kann das erfindungsgemäße Verfahren zum Erkennen einer Obstruktion verwendet werden. Weiterhin sind auch während der Obstruktion die Atemanstrengungen zu erkennen, welche z.B. für die Steuerung einer maschinellen Beatmung genutzt werden können.

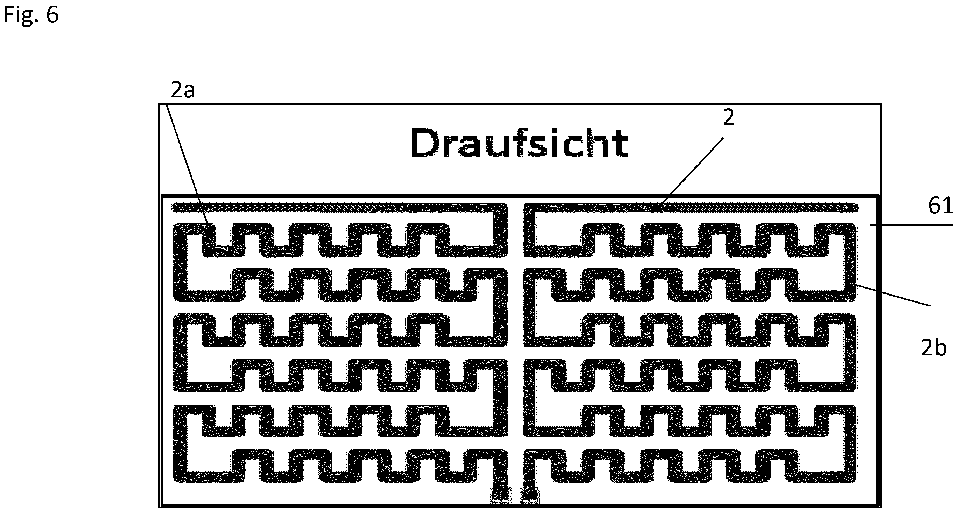

Figur 6 zeigt ein Beispiel einer Antennenstruktur 2, die auf einem Substrat 61 angeordnet ist. Das Substrat 61 kann hier eine hohe Permittivität aufweisen, wodurch eine elektrische Länge der Antenne gegenüber ihrer geometrischen Länge vergrößert wird. Die Antenne ist hier als Dipol-Antenne ausgestaltet, deren mechanische Breitedurch meanderförmiges Legen der Dipolarme 2a und 2b gegenüber ihrer elektrischen Länge (also die Länge der Leiter 2a bzw. 2b) verkürzt ist.

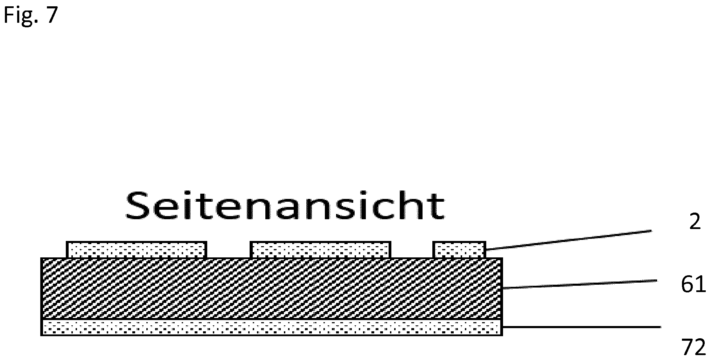

Figur 7 zeigt die in Figur 6 gezeigte Ausgestaltung der Antenne in der Seitenansicht, gesehen in Richtung parallel zur Fläche des Substrates 61. Die Antenne 2 ist in diesem Beispiel auf einem Substrat 2, angeordnet, dass auf seiner der Antenne 2 abgewandten Seite auf einer Massefläche 72 angeordnet ist. Die Massefläche kann die Antenne gegen rückseitige Störungen abschrimen.

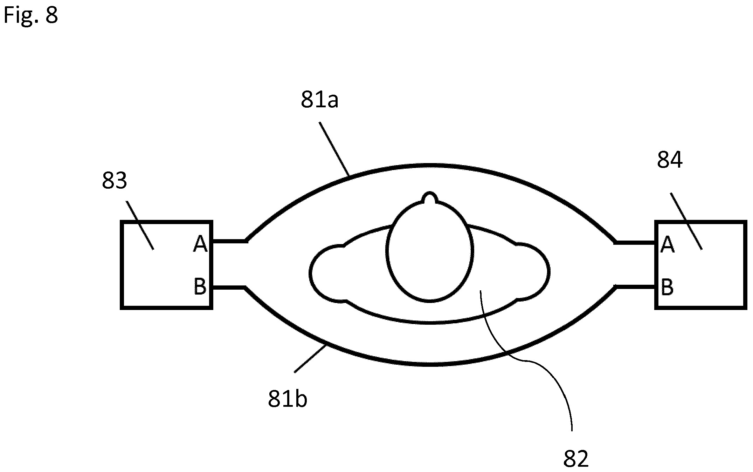

Figur 8 zeigt eine beispielhafte Ausgestaltung, bei der zwei isolierte Leiter 81a, 81b als Antennen um den Thorax des Patienten 82 gelegt sind. Dabei ist ein Leiter 81a ventral z.B. über den Brustbereich und ein Leiter 81b dorsal z.B. über den Rückenbereich geführt.

Dabei ist eine Sendestruktur 83, hier als differentielle Auslegung mit einem Anschluss A und einem Anschluss B, die gegenphasig angesteuert werden, die Empfangsstruktur 84 (ebenfalls differentiell), sowie der ventral geführte Leiter 81a und der dorsal geführte Leiter 81b gezeigt. Die Spannung kann hier zwischen den Anschlüssen A und B angelegt werden. Wird dann beispielsweise die Impedanz gemessen, so kann hieraus auf Veränderungen im Körper der Person 82 geschlossen werden.

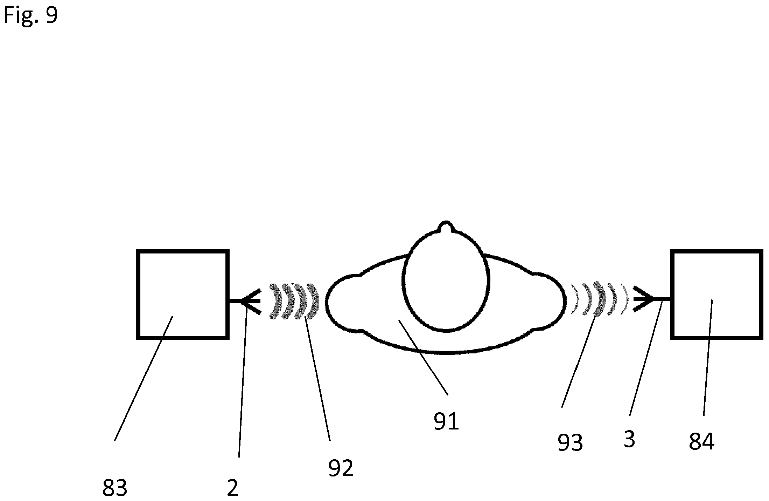

Figur 9 zeigt eine Anordnung, in der die Sende- und Empfangsantennen 2, 3 vom Körper der Person 91 beabstandet sind. Es wird hier ein Wechselfeld 92 von der Sendestruktur 83 ausgesandt und ein durch die Atmung moduliertes Feld 93 durch die Empfangsstruktur 84 empfangen. Dabei sind die Antennen vom Köper der Person 91 beabstandet. Die Wechselfelder 92, 93 durchlaufen also einen Bereich in der Luft.

Das erfindungsgemäße Verfahren ist vorteilhaft gegenüber herkömmlichen Verfahren, da die Messung direkt erfolgen kann und keine Bereinigung des Signals um eine kardiale Komponente erfolgen muss. Die kardialen Komponenten können aber zur Erhöhung der Signalqualität berücksichtigt werden. Weiterhin können Artefakte und eine Drift des Messsignals durch eine Veränderung der elektrischen Leitfähigkeit der Elektroden oder der Haut minimiert werden, da es sich hier nicht um eine Bestimmung der Leitfähigkeit handelt. Darüberhinaus ist das erfindungsgemäße Verfahren sehr arm an Bewegungsartefakten, die bei einer reinen Betrachtung von Amplitudenverhältnissen auftreten würden. Im Vergleich zu Bändern um den Brustkorb ist der Tragekomfort für den Benutzer durch eine geringe Anzahl aufgeklebter Elektroden und/oder Antennen deutlich höher. Es können auch bereits vorhandene EKG-Elektroden verwendet werden.

Die beschriebene Transmissionsmessung der Erfindung erlaubt eine klare Definition des durchmessenen Volumens, was vorteilhaft gegenüber reflektierenden Messungen ist, in denen das Volumen weniger klar definiert ist, da die Eindringtiefe von den dielektrischen Eigenschaften und damit der Struktur des durchmessenen Gewebes abhängt. Das erfindungsgemäße Verfahren erlaubt es, eine Spontanatmung während einer maschinellen Beatmung zu erkennen. Die Messungen sind intrinsisch arm an Bewegungsartefakten, so dass wenig Post-Processing und keine zusätzliche Sensorik erforderlich ist aber verwendet werden kann, um die Signalqualität zu verbessern. Der Benutzer ist verglichen mit anderen Messungen weniger eingeschränkt.

**Patentansprüche**

1. Messvorrichtung zur Bestimmung zumindest eines respiratorischen Parameters, aufweisend

   zumindest eine Sendestruktur (2), mit der ein elektromagnetisches Wechselfeld in einen Körper einstrahlbar ist,
   zumindest eine Empfangsstruktur (3), durch die das in den Körper eingestrahlte Wechselfeld empfangbar ist, nachdem es den Körper durchlaufen hat,
   einen mit der zumindest einen Sendestruktur (2) gekoppelten Signalgenerator (5), mit dem eine Wechselspannung erzeugbar ist, mit der die zumindest eine Sendestruktur (2) beaufschlagbar ist,
   eine Vergleichseinheit (9), mit der als Vergleich eine Differenz der Phase eines von der Empfangsstruktur (3) gelieferten Signals mit einer Phase der Wechselspannung zeitabhängig bildbar ist, und
   eine Auswerteeinheit (10), mit der aus einem Ergebnis des Vergleichs der Phase des von der Empfangsstruktur (3) gelieferten Signals mit der Phase der Wechselspannung zumindest ein respiratorischer Parameter bestimmbar ist,
   wobei die zumindest eine Sendestruktur (2) eine Antenne ist, und wobei die zumindest eine Empfangsstruktur (3) eine Antenne ist,
   wobei zumindest eine der Antennen der Empfangsstruktur (3) im Nahfeld zumindest einer Antenne der Sendestruktur (2) angeordnet ist, wobei ein Abstand zwischen den Antennen kleiner oder gleich der vierfachen Wellenlänge des eingestrahlten Signals ist,
   wobei der zumindest eine respiratorische Parameter zumindest eines ausgewählt aus den Folgenden Größen ist:

      ob eine Inspiration oder eine Exspiration stattfindet,
      ein Beginn einer Inspiration oder Exspiration ,
      ein Ende einer Inspiration oder Exspiration,
      ein Verlauf einer Inspiration und/oder Exspiration
      wobei die Sende- und/oder Empfangsstruktur in einer Auflagefläche für einen Patienten oder in einem Seitenelement eines Patientenbettes angeordnet ist.

2. Messvorrichtung nach dem vorhergehenden Anspruch, wobei die Wechselspannung mit variierbarer und/oder variierender Frequenz erzeugbar ist.

3. Messvorrichtung nach einem der vorhergehenden Ansprüche,

   wobei die zumindest eine Sendestruktur (2) über ein erstes Kabel mit vorgegebener Wellenimpedanz mit dem Signalgenerator (5) verbunden ist und über einen ersten Widerstand gespeist wird, dessen Wert gleich der Wellenimpedanz des ersten Kabels ist,
   und wobei die Empfangsstruktur (3) über ein zweites Kabel mit vorgegebener Wellenimpedanz mit der Vergleichseinheit (9) verbunden ist und über einen zweiten Widerstand terminiert wird, dessen Wert gleich der Wellenimpedanz des zweiten Kabels ist,
   wobei vorzugsweise der erste und der zweite Widerstand sowie die Wellenimpedanz des ersten und zweiten Kabels 50 Ohm beträgt.

4. Messvorrichtung nach einem der vorhergehenden Ansprüche, wobei der Signalgenerator (5) eingerichtet ist, die Wechselspannung mit einer Frequenz von größer oder gleich 10 MHz, vorzugsweise größer oder gleich 30 MHz, vorzugsweise größer oder gleich 100 MHz und/oder kleiner oder gleich 1000 MHz, vorzugsweise kleiner oder gleich 500 MHz, vorzugsweise kleiner oder gleich 300 MHz zu erzeugen.

5.  Messvorrichtung nach einem der vorhergehenden Ansprüche, wobei die Messvorrichtung drei, vier oder mehr als vier der Sendestrukturen (2) aufweist und/oder drei, vier oder mehr als vier der Empfangsstrukturen (3) aufweist.

6.  Messvorrichtung nach einem der beiden vorhergehenden Ansprüche, wobei die zumindest eine Sende- und/oder Empfangsantenne eine meanderförmige Struktur (2a, 2b) aufweist, wobei vorteilhafterweise die zumindest eine Sende- und/oder Empfangsantenne auf einem Substrat mit erhöhter Permittivität aufgebracht ist.

7.  Verfahren zum Messen zumindest eines respiratorischen Parameters, wobei

    über zumindest eine Sendestruktur (2) ein elektromagnetisches Wechselfeld in einen Körper eingestrahlt wird, indem durch einen Signalgenerator (5) eine Wechselspannung erzeugt wird, mit der die zumindest eine Sendestruktur (2) beaufschlagt wird,
    das in den Körper eingestrahlte Wechselfeld durch zumindest eine Empfangsstruktur (3) empfangen wird, nachdem es den Körper durchlaufen hat,
    in einem Vergleichsschritt als Vergleich eine Differenz der Phase eines von der zumindest einen Empfangsstruktur (3) gelieferten Signals mit einer Phase der Wechselspannung zeitabhängig gebildet wird und aus einem Ergebnis des Vergleichsschritts zumindest ein respiratorischer Parameter bestimmt wird,
    wobei die zumindest eine Sendestruktur (2) eine Antenne ist, und wobei die zumindest eine Empfangsstruktur (3) eine Antenne ist, wobei zumindest eine der Antennen der Empfangsstruktur (3) im Nahfeld zumindest einer Antenne der Sendestruktur (2) angeordnet ist, wobei ein Abstand zwischen den Antennen kleiner oder gleich der vierfachen Wellenlänge des eingestrahlten Signals ist,
    wobei der zumindest eine respiratorische Parameter zumindest eines ausgewählt aus den Folgenden Größen ist:

    ob eine Inspiration oder eine Exspiration stattfindet,
    ein Beginn einer Inspiration oder Exspiration ,
    ein Ende einer Inspiration oder Exspiration,
    ein Verlauf einer Inspiration und/oder Exspiration,
    wobei die Sende- und/oder Empfangsstruktur in einer Auflagefläche für einen Patienten oder in einem Seitenelement eines Patientenbettes angeordnet ist.

8.  Verfahren nach dem vorhergehenden Anspruch,

    wobei die zumindest eine Sendestruktur (2) auf einem Thorax und/oder einem Abdomen des Körpers angeordnet wird, und
    die zumindest eine Empfangsstruktur (3) auf dem Thorax und/oder dem Abdomen angebracht wird.

9.  Verfahren nach dem vorhergehenden Anspruch, wobei die Sendestruktur (2) und die Empfangsstruktur (3) lateral am Abdomen und/oder am Thorax angebracht werden.

10. Verfahren nach einem der Ansprüche 7 bis 9 wobei aus dem Vergleich der Phase des von der zumindest einen Empfangsstruktur (3) gelieferten Signals mit der Phase der Wechselspannung ein maximaler Phasenhub bestimmt wird und aus dem maximalen Phasenhub als der zumindest eine respiratorische Parameter ein Atemzugvolumen bestimmt wird.

11. Verfahren nach einem der Ansprüche 7 bis 10 wobei eine Ableitung der Differenz zwischen der Phase des von der zumindest einen Empfangsstruktur (3) gelieferten Signals und der Phase Wechselspannung bestimmt wird und aus der Ableitung als respiratorischer Parameter bestimmt wird, ob eine Inspiration oder Exspiration stattfindet.

12. Verfahren nach einem der Ansprüche 7 bis 11,
    wobei die Wechselspannung, vorzugsweise nacheinander, mit einer Mehrzahl unterschiedlicher Frequenzen erzeugt wird und der zumindest eine respiratorische Parameter aus einem Vergleich der Phase des von der zumindest einen Empfangsstruktur (3) gelieferten Signals mit der Phase der Wechselspannung bei einer der Frequenzen bestimmt wird, bei der unter allen der Mehrzahl an Frequenzen eine Kopplung zwischen der Sendestruktur (2) und der Empfangsstruktur (3) maximal ist oder eine Amplitude des von der Empfangsstruktur (3) gelieferten Signals maximal ist oder sich über den Verlauf eines Atemzyklus eine maximale Veränderung der Differenz zwischen der Phase des von der Empfangsstruktur (3) gelieferten Signals und der Phase Wechselspannung ergibt.

13. Verfahren nach einem der Ansprüche 7 bis 12,

wobei die Wechselspannung, vorzugsweise zeitlich nacheinander, mit einer Mehrzahl unterschiedlicher Frequenzen erzeugt wird und der zumindest eine respiratorische Parameter aus einem Vergleich der Phase des von der Empfangsstruktur (3) gelieferten Signals mit der Phase der Wechselspannung bei zumindest zweien der angelegten Frequenzen bestimmt wird.

14. Verfahren nach einem der Ansprüche 7 bis 13, wobei als der zumindest eine respiratorischer Parameter ein eine Blockade eines Atemweges und/oder eine Atemanstrengung gegen eine maschinelle Beatmung bestimmt wird.

15. Verfahren nach einem der Ansprüche 7 bis 14, wobei eine zeitliche Änderung einer Differenz zwischen der Phase des von der Empfangsstruktur (3) gelieferten Signals und der Phase der Wechselspannung in Abhängigkeit von der Zeit dargestellt wird.

16. Verfahren nach einem der Ansprüche 7 bis 15, wobei zumindest eine weitere Elektrode und/oder zumindest eine weitere Messfrequenz ausgewertet wird, um zumindest eine Störgröße, vorzugsweise einen Einfluss eines Herzschlags und/oder einer Bewegung des Körpers, herauszurechnen.

17. Verfahren nach einem der Ansprüche 7 bis 16, wobei zumindest eine Störgröße aus einem in dem Verfahren bestimmten respiratorischen Parameter, vorzugsweise mittels eines Frequenzfilters, eines adaptiven Filters, eines Glättungsfilters und/oder einer Ableitung, herausgerechnet wird.

18. Verfahren nach einem der Ansprüche 7 bis 17, wobei das Verfahren ein nicht-diagnostisches Verfahren ist.

19. Beatmungsgerät, aufweisend eine Messvorrichtung nach einem der Ansprüche 1 bis 6, wobei das Beatmungsgerät eingerichtet ist, ein Verfahren nach einem der Ansprüche 7 bis 18 auszuführen und die Beatmung anhand des zumindest einen respiratorischen Parameters zu steuern.

20. Gerät zur Injektion eines Kontrastmittels für bildgebende Verfahren, aufweisend eine Messvorrichtung nach einem der Ansprüche 1 bis 6, wobei das Gerät eingerichtet ist, ein Verfahren nach einem der Ansprüche 7 bis 18 auszuführen, um die Injektion des Kontrastmittels als Funktion des respiratorischen Parameters auszuführen.

21. Gerät zur Bildgebung, aufweisend eine Messvorrichtung nach einem der Ansprüche 1 bis 6, wobei das Gerät eingerichtet ist, ein Verfahren nach einem der Ansprüche 7 bis 18 auszuführen, um eine Bildaufnahme nur bei definierten respiratorischen Parametern durchzuführen oder/und gewonnene Bilddaten nur bei definierten respiratorischen Parametern zu verwenden oder/und eine Verrechnung von gewonnenen Bilddaten als Funktion der respiratorischen Parameter durchzuführen.

**Claims**

1. A measuring device for determining at least one respiratory parameter, comprising:

   at least one transmitting structure (2) with which an electromagnetic alternating field can be irradiated into a body,
   at least one receiving structure (3) through which the alternating field irradiated into the body can be received after it has passed through the body,
   a signal generator (5), which is coupled to the at least one transmitting structure (2) and with which an AC voltage can be generated that can be applied to the at least one transmitting structure (2),
   a comparison unit (9), with which as a comparison a difference of the phase of a signal supplied from the receiving structure (3) to a phase of the AC voltage can be formed as a function of time; and
   an evaluation unit (10), with which at least one respiratory parameter can be determined from a result of the comparison of the phase of the signal supplied from the receiving structure (3) to the phase of the AC voltage, wherein the at least one transmitting structure (2) is an antenna, and wherein the at least one receiving structure (3) is an antenna,
   wherein at least one of the antennas of the receiving structure (3) is arranged in the near field of at least one antenna of the transmitting structure (2),
   wherein a distance between the antennas is less than or equal to four times the wavelength of the irradiated signal,
   wherein the at least one respiratory parameter is at least one selected from the following quantities:

whether an inspiration or an expiration is taking place,
a beginning of an inspiration or expiration,
an end of an inspiration or expiration,
a course of an inspiration and/or expiration
wherein the transmitting and/or receiving structure is arranged in a support surface for a patient or in a side element of a patient bed.

2. The measuring device according to the preceding claim, wherein the AC voltage can be generated with variable and/or varying frequency.

3. The measuring device according to any one of the preceding claims, wherein the at least one transmitting structure (2) is connected to the signal generator (5) via a first cable having a predefined wave impedance and is fed via a first resistance, the value of which is equal to the wave impedance of the first cable,

and wherein the receiving structure (3) is connected to the comparison unit (9) via a second cable having a predefined wave impedance and is terminated via a second resistance, the value of which is equal to the wave impedance of the second cable,
the first and second resistances as well as the wave impedance of the first and second cables preferably being 50 ohms.

4. The measuring device according to any one of the preceding claims, wherein the signal generator (5) is configured to generate the AC voltage with a frequency of greater than or equal to 10 MHz, preferably greater than or equal to 30 MHz, preferably greater than or equal to 100 MHz, and/or smaller than or equal to 1000 MHz, preferably smaller than or equal to 500 MHz, and preferably smaller than or equal to 300 MHz.

5. The measuring device according to any one of the preceding claims, wherein the measuring device comprises three, four or more than four of the transmitting structures (2) and/or three, four or more than four of the receiving structures (3).

6. The measuring device according to any one of the two preceding claims, wherein the at least one transmitting and/or receiving antenna has a meander-shaped structure (2a, 2b), the at least one transmitting and/or receiving antenna advantageously being applied to a substrate having increased permittivity.

7. A method for determining at least one respiratory parameter,
wherein

an electromagnetic alternating field is irradiated, via at least one transmitting structure (2), into a body by generating an AC voltage with a signal generator (5), which AC voltage is applied to the at least one transmitting structure (2),
the alternating field irradiated into the body is received by at least one receiving structure (3) after having passed through the body,
in a comparison step, as a comparison a difference of the phase of a signal supplied from the at least one receiving structure (3) to a phase of the AC voltage is then formed as a function of time, and at least one respiratory parameter is determined from a result of the comparison step,
wherein the at least one transmitting structure (2) is an antenna, and wherein the at least one receiving structure (3) is an antenna,
wherein at least one of the antennas of the receiving structure (3) is arranged in the near field of at least one antenna of the transmitting structure (2), wherein a distance between the antennas is less than or equal to four times the wavelength of the irradiated signal,
wherein the at least one respiratory parameter is at least one selected from the following quantities:

whether an inspiration or an expiration is taking place,
a beginning of an inspiration or expiration,
an end of an inspiration or expiration,
a course of an inspiration and/or expiration,
wherein the transmitting and/or receiving structure is arranged in a support surface for a patient or in a side element of a patient bed.

8. The method according to the preceding claim,

   wherein the at least one transmitting structure (2) is arranged on a thorax and/or an abdomen of the body, and the at least one receiving structure (3) is attached to the thorax and/or the abdomen.

9. The method according to the preceding claim, wherein the transmitting structure (2) and the receiving structure (3) are attached laterally at the abdomen and/or at the thorax.

10. The method according to any one of claims 7 to 9, wherein a maximum phase deviation is determined from the comparison of the phase of the signal supplied by the at least one receiving structure (3) to the phase of the AC voltage, and a volume of a breath of air is determined as the at least one respiratory parameter from the maximum phase deviation.

11. The method according to any one of claims 7 to 10, wherein a derivative of the difference between the phase of the signal supplied by the at least one receiving structure (3) to the phase of the AC voltage is determined, and whether inspiration or expiration is taking place is determined as the respiratory parameter from the derivative.

12. The method according to any of claims 7 to 11, wherein the AC voltage is generated, preferably successively, having a plurality of different frequencies, and the at least one respiratory parameter is determined from a comparison of the phase of the signal supplied by the at least one receiving structure (3) to the phase of the AC voltage at one of the frequencies at which, among all of the plurality of frequencies, the coupling between the transmitting structure (2) and the receiving structure (3) is maximal, or an amplitude of the signal supplied by the receiving structure (3) is maximal, or a maximum change in the difference between the phase of the signal supplied by the receiving structure (3) and the phase of the AC voltage results over the course of a breathing cycle.

13. The method according to any of claims 7 to 12, wherein the AC voltage is generated, preferably in chronological succession, using a plurality of different frequencies, and the at least one respiratory parameter is determined from a comparison of the phase of the signal supplied by the receiving structure (3) to the phase of the AC voltage at at least two of the applied frequencies.

14. The method according to any of claims 7 to 13, wherein a blockage of the respiratory tract and/or labored breathing against mechanical ventilation is determined as the at least one respiratory parameter.

15. The method according to any one of claims 7 to 14, wherein a temporal change in a difference between the phase of the signal supplied by the receiving structure (3) and the phase of the AC voltage is represented as a function of time.

16. The method according to any one of claims 7 to 15, wherein at least one further electrode and/or at least one further measuring frequency is evaluated so as to remove at least one disturbance quantity, preferably an influence of a heart beat and/or a movement of the body, from the computation.

17. The method according to any one of claims 7 to 16, wherein at least one disturbance quantity is removed from the computation of a respiratory parameter determined in the method, for example by means of a frequency filter, an adaptive filter, a smoothing filter and/or a derivative.

18. The method according to any one of claims 7 to 17, wherein the method is a non-diagnostic method.

19. A respiratory apparatus comprising a measuring device according to any one of claims 1 to 6, wherein the respiratory apparatus is configured to carry out a method according to any one of claims 7 to 18, and to control the respiration based on the at least one respiratory parameter.

20. An apparatus for injecting a contrast agent for imaging processes, comprising a measuring device according to any one of claims 1 to 6, wherein the apparatus is configured to carry out a method according to any one of claims 7 to 18 so as to carry out the injection of the contrast agent as a function of the respiratory parameter.

21. An apparatus for imaging, comprising a measuring device according to any one of claims 1 to 6, wherein the apparatus is configured to carry out a method according to any one of claims 7 to 18 so as to only record images at defined respiratory parameters and/or so as to only use collected image data at defined respiratory parameters and/or so as to offset collected image data in the computation as a function of the respiratory parameters.

**Revendications**

1. Dispositif de mesure pour la détermination d'au moins un paramètre respiratoire, comprenant

au moins une structure émettrice (2) avec laquelle un champ électromagnétique alternatif peut être envoyé dans un corps,
au moins une structure réceptrice (3), grâce à laquelle le champ alternatif envoyé dans le corps peut être reçu après avoir traversé le corps,
un générateur de signaux (5), couplé avec l'au moins une structure émettrice (2), avec lequel une tension alternative peut être générée, avec laquelle l'au moins une structure émettrice (2) peut être alimentée,
une unité de comparaison (9) avec laquelle, pour une comparaison, une différence entre la phase d'un signal délivré par la structure réceptrice (3) et une phase de la tension alternative peut être formée en fonction du temps et
une unité d'analyse (10) avec laquelle, à partir d'un résultat de la comparaison de la phase du signal délivré par la structure réceptrice (3) avec la phase de la tension alternative, au moins un paramètre respiratoire peut être déterminé,
dans lequel l'au moins une structure émettrice (2) est une antenne et dans lequel l'au moins une structure réceptrice (3) est une antenne,
dans lequel au moins une des antennes de la structure réceptrice (3) est disposée à proximité d'au moins une antenne de la structure émettrice (2),
dans lequel une distance entre les antennes est inférieure ou égale au quadruple de la longueur d'onde du signal émis,
dans lequel l'au moins un paramètre respiratoire est au moins une grandeur sélectionnée parmi les grandeurs suivantes :

si une inspiration ou une expiration a lieu,
un début d'une inspiration ou d'expiration,
une fin d'inspiration ou d'expiration,
un tracé d'une inspiration et/ou d'expiration
dans lequel la structure émettrice et/ou réceptrice est disposée dans une surface d'appui pour un patient ou dans un élément latéral d'un lit de patient.

2. Dispositif de mesure selon la revendication précédente, dans lequel la tension alternative peut être générée avec une fréquence pouvant être modifiée et/ou variable.

3. Dispositif de mesure selon l'une des revendications précédentes, dans lequel l'au moins une structure émettrice (2) est reliée, par l'intermédiaire d'un premier câble avec une impédance d'onde prédéterminée, avec le générateur de signaux (5) et est alimentée par l'intermédiaire d'une première résistance, dont la valeur est égale à l'impédance d'onde du premier câble,

et dans lequel la structure réceptrice (3) est reliée, par l'intermédiaire d'un deuxième câble avec une impédance d'onde prédéterminée, avec l'unité de comparaison (9) et est terminée par l'intermédiaire d'une deuxième résistance, dont la valeur est égale à l'impédance d'onde du deuxième câble,
dans lequel, de préférence, les première et deuxième résistances ainsi que l'impédance d'onde des premier et deuxième câbles est de 50 Ohm.

4. Dispositif de mesure selon l'une des revendications précédentes, dans lequel le générateur de signaux (5) est conçu pour générer la tension alternative avec une fréquence supérieure ou égale à 10 MHz, de préférence supérieure ou égale à 30 MHz, de préférence supérieure ou égale à 100 MHz et/ou inférieure ou égale à 1000 MHz, de préférence inférieure ou égale à 500 MHz, de préférence inférieure ou égale à 300 MHz.

5. Dispositif de mesure selon l'une des revendications précédentes, dans lequel le dispositif de mesure comprend trois, quatre ou plus de quatre structures émettrices (2) et/ou trois, quatre ou plus de quatre structures réceptrices (3).

**6.** Dispositif de mesure selon l'une des deux revendications précédentes, dans lequel l'au moins une antenne émettrice et/ou réceptrice présente une structure en forme de méandres (2a, 2b), dans lequel, de manière avantageuse, l'au moins une antenne émettrice et/ou réceptrice est appliquée sur un substrat avec une permittivité augmentée.

**7.** Procédé de mesure d'au moins un paramètre respiratoire,
dans lequel

par l'intermédiaire d'au moins une structure émettrice (2), un champ électromagnétique alternatif est envoyé dans un corps, grâce au fait que, à l'aide d'un générateur de signaux (5), une tension alternative est générée, avec laquelle l'au moins une structure émettrice (2) est alimentée,
le champ alternatif envoyé dans le corps est reçu par au moins une structure réceptrice (3) après avoir traversé le corps,
dans une étape de comparaison, pour effectuer une comparaison, une différence entre la phase d'un signal délivré par l'au moins une structure réceptrice (3) et une phase de la tension alternative est formée en fonction du temps et, à partir d'un résultat de l'étape de comparaison, au moins un paramètre respiratoire est déterminé,
dans lequel l'au moins une structure émettrice (2) est une antenne et dans lequel l'au moins une structure réceptrice (3) est une antenne,
dans lequel au moins une des antennes de la structure réceptrice (3) est disposée à proximité d'au moins une antenne de la structure émettrice (2),
dans lequel une distance entre les antennes est inférieure ou égale au quadruple de la longueur d'onde du signal émis,
dans lequel l'au moins un paramètre respiratoire est au moins une grandeur sélectionnée parmi les grandeurs suivantes :

si une inspiration ou une expiration a lieu,
un début d'une inspiration ou d'expiration,
une fin d'inspiration ou d'expiration,
un tracé d'une inspiration et/ou d'expiration
dans lequel la structure émettrice et/ou réceptrice est disposée dans une surface d'appui pour un patient ou dans un élément latéral d'un lit de patient.

**8.** Procédé selon la revendication précédente,

dans lequel l'au moins une structure émettrice (2) est disposée sur un thorax et/ou un abdomen du corps et l'au moins une structure réceptrice (3) est posée sur le thorax et/ou l'abdomen.

**9.** Procédé selon la revendication précédente, dans lequel la structure émettrice (2) et la structure réceptrice (3) sont posées latéralement sur l'abdomen et/ou sur le thorax.

**10.** Procédé selon l'une des revendications 7 à 9,
dans lequel, à partir de la comparaison entre la phase du signal délivré par la structure réceptrice (3) et la phase de la tension alternative, un déphasage maximal est déterminé et, à partir du déphasage maximal, un volume respiratoire est déterminé en tant que l'au moins un paramètre respiratoire.

**11.** Procédé selon l'une des revendications 7 à 10, dans lequel une dérivée de la différence entre la phase du signal délivré par l'au moins une structure réceptrice (3) et la phase de la tension alternative est déterminé et, à partir de la dérivée, il est déterminé, en tant que paramètre respiratoire, si une inspiration ou une expiration a lieu.

**12.** Procédé selon l'une des revendications 7 à 11,
dans lequel la tension alternative est générée, de préférence successivement, avec une pluralité de fréquences différentes et l'au moins un paramètre respiratoire est déterminé à partir d'une comparaison entre la phase du signal délivré par l'au moins une structure réceptrice (3) et la phase de la tension alternative pour une des fréquences, pour laquelle, parmi toutes les fréquences de la pluralité de fréquences, un couplage entre la structure émettrice (2) et la structure réceptrice (3) est maximal ou une amplitude du signal délivré par la structure réceptrice (3) est maximale ou, sur le tracé d'un cycle respiratoire, une variation maximale de la différence entre la phase du signal délivré par la structure réceptrice (3) et la phase de la tension alternative est obtenue.

**13.** Procédé selon l'une des revendications 7 à 12,

dans lequel la tension alternative est générée, de préférence successivement dans le temps, avec une pluralité de fréquences différentes et l'au moins un paramètre respiratoire est déterminé à partir d'une comparaison entre la phase du signal délivré par la structure réceptrice (3) et la phase de la tension alternative pour au moins deux des fréquences appliquées.

14. Procédé selon l'une des revendications 7 à 13, dans lequel, en tant qu'au moins un paramètre respiratoire, un blocage d'une voie respiratoire et/ou un effort respiratoire contre une respiration artificielle est déterminé.

15. Procédé selon l'une des revendications 7 à 14, dans lequel une variation dans le temps d'une différence entre la phase du signal délivré par la structure réceptrice (3) et la phase de la tension alternative est représentée en fonction du temps.

16. Procédé selon l'une des revendications 7 à 15, dans lequel au moins une autre électrode et/ou au moins une autre fréquence de mesure est analysée, afin de calculer au moins une grandeur parasite, de préférence l'influence d'un rythme cardiaque et/ou d'un déplacement du corps.

17. Procédé selon l'une des revendications 7 à 16, dans lequel au moins une grandeur parasite est calculée à partir d'un paramètre respiratoire déterminé dans le procédé, de préférence au moyen d'un filtre de fréquence, d'un filtre adaptatif, d'un filtre de lissage et/ou d'une dérivée.

18. Procédé selon l'une des revendications 7 à 17, dans lequel le procédé n'est pas un procédé de diagnostic.

19. Appareil respiratoire, comprenant un dispositif de mesure selon l'une des revendications 1 à 6, dans lequel l'appareil respiratoire est conçu pour exécuter un procédé selon l'une des revendications 7 à 18 et contrôler la respiration à l'aide de l'au moins un paramètre respiratoire.

20. Appareil pour l'injection d'un produit de contraste pour un procédé d'imagerie, comprenant un dispositif de mesure selon l'une des revendications 1 à 6, dans lequel l'appareil est conçu pour exécuter un procédé selon l'une des revendications 7 à 18 afin d'exécuter l'injection du produit de contraste en fonction du paramètre respiratoire.

21. Appareil d'imagerie comprenant un dispositif de mesure selon l'une des revendications 1 à 6, dans lequel l'appareil est conçu pour exécuter un procédé selon l'une des revendications 7 à 18 afin d'effectuer une prise de vue uniquement dans le cas de paramètres respiratoires définis et/ou afin d'utiliser les données d'images obtenues uniquement dans le cas de paramètres respiratoires définis et/ou d'effectuer un calcul des données d'images obtenues en fonction des paramètres respiratoires.

Fig. 1

Fig. 2

Fig. 3

EP 3 809 964 B1

**Fig. 4**

"Körper"

Signal-generator

Auswerte-einheit

**A**
Phasenversatz Phi der transmittierten Welle (des Signals)

Gesendet

Empfangen

t

**B**
Inspiration

Beginn der Exspiration

Exspiration

Phi

ΔPhi (Veränderung der Phase in einem z.B. Atemzyklus)

t

**C**
Verlauf des beatmeten Volumens

ΔPhi

t

EP 3 809 964 B1

Fig. 5

Obstruktion Atmung

EP 3 809 964 B1

22

Fig. 6

Draufsicht

Fig. 7

**Seitenansicht**

- 2
- 61
- 72

Fig. 8

EP 3 809 964 B1

EP 3 809 964 B1

Fig. 9

83

2

92

91

93

3

84

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20130226527 A1
- US 20170172425 A1
- US 4958638 A
- US 20160374622 A1
- US 20080077015 A1
- US 4926868 A
- US 20120073574 A1
- US 20160174926 A1
- US 20150031979 A1

**In der Beschreibung aufgeführte Nicht-Patentliteratur**

- **PETKIE T DOUGLAS.** Millimeter-wave radar systems for biometric applications. *SPIE, PO BOX 10 Bellingham WA 98227-0010 USA,* 17. September 2009, vol. 7485, 748502
- **H. WHEELER.** The Radiansphere around a Small Antenna. *Proc. IRE,* 1959, vol. 47 (8), 1325-1331